(19) Europäisches Patentamt
European Patent Office
Office européen des brevets

(11) Publication number: **0 241 846 B1**

# EUROPEAN PATENT SPECIFICATION

(45) Date of publication of patent specification: **16.10.91**  (51) Int. Cl.⁵: **A61F 2/36**

(21) Application number: **87105131.4**

(22) Date of filing: **07.04.87**

(54) **Femoral prosthesis for total hip replacement.**

(30) Priority: **15.04.86 US 852440**

(43) Date of publication of application:
**21.10.87 Bulletin 87/43**

(45) Publication of the grant of the patent:
**16.10.91 Bulletin 91/42**

(84) Designated Contracting States:
**BE DE FR GB NL SE**

(56) References cited:
EP-A- 0 128 036    EP-A- 0 163 013
DE-A- 3 003 050    FR-A- 2 215 927
US-A- 4 536 894    US-A- 4 550 448
US-A- 4 608 055

(73) Proprietor: **NEW YORK SOCIETY FOR THE RE-
LIEF OF THE RUPTURED AND CRIPPLED
MAINTAINING THE HOSPITAL FOR SPECIAL
SURGERY
The Hospital for Special Surgery 535 East
70th Street
New York, N.Y. 10021(US)**

(72) Inventor: **Ranawat, Chitranjan S.
3 Buckingham Drive
Alpine, New Jersey 07620(US)**
Inventor: **Burstein, Albert H.
7 Farm Hill Road
Stamford, Connecticut 06902(US)**
Inventor: **Bartel, Donald L.
69 Yellow Barn Road
Freeville, N.Y. 13068(US)**

(74) Representative: **Goetz, Rupert, Dipl.-Ing. et al
Wuesthoff & Wuesthoff Patent- und Recht-
sanwälte Schweigerstrasse 2
W-8000 München 90(DE)**

## Description

This invention relates to a femoral prosthesis according to the preamble of claim 1.

In total hip replacement the head and most of the neck of the proximal femur are removed and a femoral prosthesis is implanted. The conventional approach in designing a femoral prosthesis has assumed that loads should be transferred between the prosthesis and the femur over the entire length of the part of the prosthesis that is received within the femur, which includes, of course, the stem that extends a relatively large distance into the femoral shaft. The distribution of the load transfer depends on the specific design of the prosthesis and the type of fixation.

In prostheses that are implanted with bone cement (polymethyl methacrylate), the loads are transferred from the prosthesis through the cement to the endosteal surface of the bone throughout the entire cement layer, but the distribution of stress is non-uniform, though it can be predicted using stress analysis techniques such as the finite element method. In prostheses that are implanted without bone cement, sintered porous metal coatings on the prosthesis promote ingrowth of bone for biological retention, and the distribution of the load transfer from the prosthesis to the endosteal surface of the bone depends upon apposition of the bone, the extent of bone ingrowth and the design of the prosthesis. In presently known designs of non-cemented femoral prostheses the apposition and, hence, the regions where load transfer will occur are unpredictable.

Bone remodels according to the loads applied to it, and the long term stress distribution in the bone is, therefore, of great importance. A beneficial effect of the remodeling property of bone is, of course, that the structural characteristics of regions of the bone that are loaded by the prosthesis and by the tendons at the hip joint are likely to be enhanced. Conversely, regions of the bone where the loads are decreased deteriorate through loss of bone structure. It is, therefore, important that a prosthesis provide for load transfer that is predictable and that minimizes the possibility of unloading of the bone.

From EP-A 0 128 036 a femoral hip prosthesis has been known which comprises a stem component generally divided into a proximal portion and a substantially longer distal portion. The characteristics of the stem provide a glove fit when inserted into a prepared intramedullery canal in the femur.

EP-A 0 163 013 discloses a femoral component for a hip joint prosthesis having a stem including a rounded medial aspect and a lateral aspect. To prevent loading the femur in the region of the calcar the lateral aspect is formed linear.

Inherent in known designs of femoral hip joint prostheses of both the cemented and non-cemented types is some degree of unloading of the bone in the region of the stem of the prosthesis, inasmuch as the stem is coupled to the bone to a lesser or greater extent, depending on the design. When the bone bends, the stem has to bend, which produces stress in the stem, stress (load) that would otherwise be borne by the bone. Bending of the stem also alters the load transfer between the bone and the prosthesis at the proximal region, tending to unload the proximal medial aspect of the femur, a region where the bone is usually under a large axial compressive load, and load the proximal lateral aspect, a region where the bone is under tension. Bone resorption at the proximal femur reduces load transfer in that region and increases the load transfer in the stem. Loosening of the implant and stem fracture can result.

It is an object of the invention to provide a prosthesis that offers predictable load transfer and minimizes the possibility of unloading of the bone.

According to the present invention, this object is met by the features of claim 1.

The inventive femoral prosthesis provides loading in the intertrochanteric region of the femur that produces stress levels similar to those occurring in a normal anatomical femur. Further, it provides predictable load transfer by establishing specific, known regions of contact between the prosthesis and endosteal surfaces of the femur and minimizing contact other than in those regions. Third, transfer of loads between the stem and bone in the axial direction is minimized so that the proximal portion of the femoral shaft continues to be loaded in approximately the same region as it is in a normal anatomical hip joint. The foregoing principles ensure to a maximum degree possible that the prosthesis will distribute loads to the femur in a manner that will preserve bone structure. In addition it is intended that stresses in the stem of the prosthesis be minimized.

A femoral component, according to the present invention, is adapted to be implanted in the proximal portion of a femur for biological retention without cement and has a proximal portion adapted to be received in the intertrochanteric region of the femur, a neck portion extending superiorly from the proximal portion obliquely both medially and anteriorly and adapted to be joined to a femoral head element, and a stem portion smoothly merging with and extending inferiorly from the proximal portion. The invention is characterized in that the proximal portion has anterior and posterior aspects that converge both medially and inferiorly and intersect a rounded medial aspect oriented obliquely to the stem portion both anteriorly and medially, said aspects defining a generally wedge-shaped body adapted to engage the endosteal surfaces of the femur in the intertrochanteric region of the femur such that substantially all loads are transmitted from the component to the bone in that region and load transfers through other surfaces, especially the stem, are minimized.

In a preferred embodiment the anterior and posterior aspects of the proximal portion are planar surfaces, and a recess extends continuously along the medial portions of the anterior and posterior aspects and around the medial aspect, the recess containing a porous medium adapted to accept bone ingrowth at the calcar region of the femur for biological retention.

For a better understanding of the invention, reference may be made to the following description of an exemplary embodiment, taken in conjunction with the accompanying drawings.

Description of the Drawings

Fig. 1 is a front elevational view of the embodiment taken orthogonally to a medial-lateral plane of the prosthesis that includes the stem axis;

Fig. 2 is a side elevational view of the lateral side taken orthogonally to an anterior-posterior plane that includes the stem axis;

Fig. 3 is a top plan view looking directly down the stem axis;

Fig. 4 is a front elevational view taken orthogonally to a plane defined by the axes of the stem and the proximal portion ("cone"), as indicated by the arrowed lines 4-4 adjacent Fig. 3;

Fig. 5 is an elevational view taken orthogonally to a medial-lateral plane that includes the cone axis, as indicated by the arrowed lines 5-5 adjacent Fig. 2;

Fig. 6 is a top plan view looking down the cone axis;

Fig. 7 is a front elevational view taken orthogonally to a plane defined by the axes of the neck and cone, as indicated by the arrowed lines 7-7 adjacent Fig. 6; and

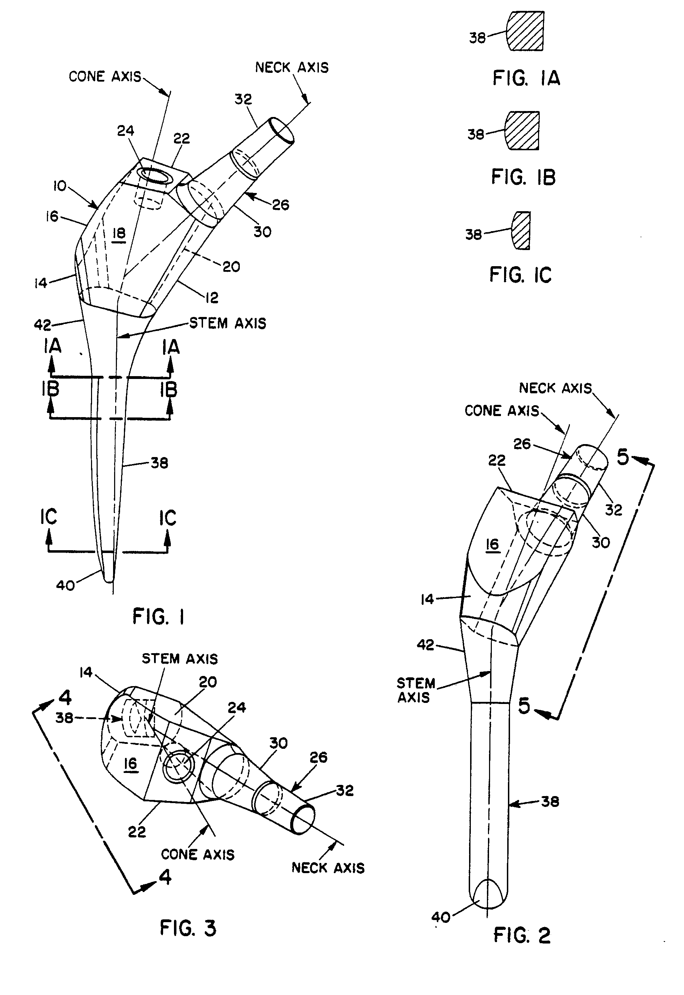

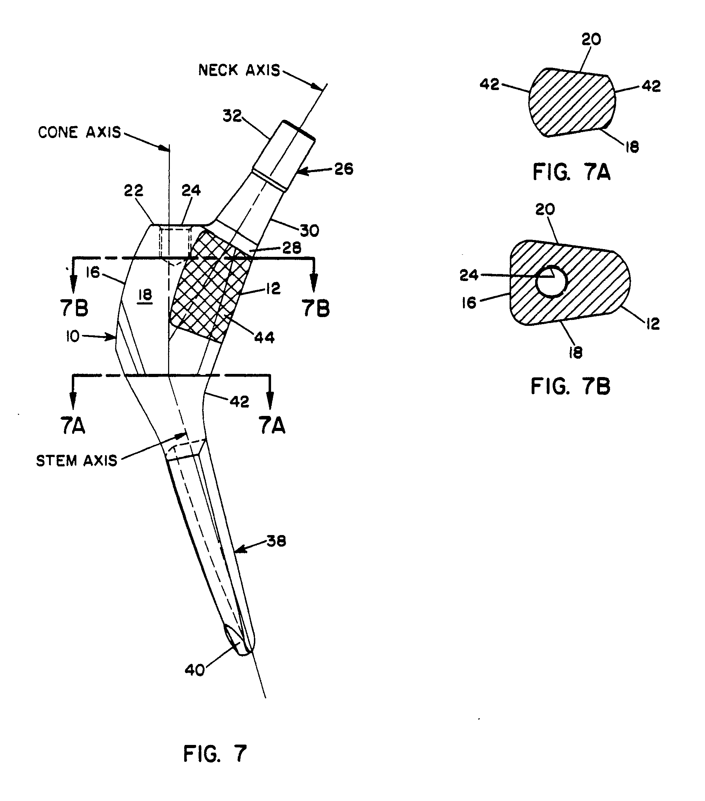

Figs. 1A, 1B, 1C, 7A and 7B are cross-sectional views taken along planes represented by the correspondingly labelled, arrowed lines in the Figs. 1 and 7 and in the direction of the arrows.

Description of the Embodiment

The drawings show a component for a right hip. The unique design of the invention requires individual right and left parts, unlike many designs, in which a single part can be used in either hip.

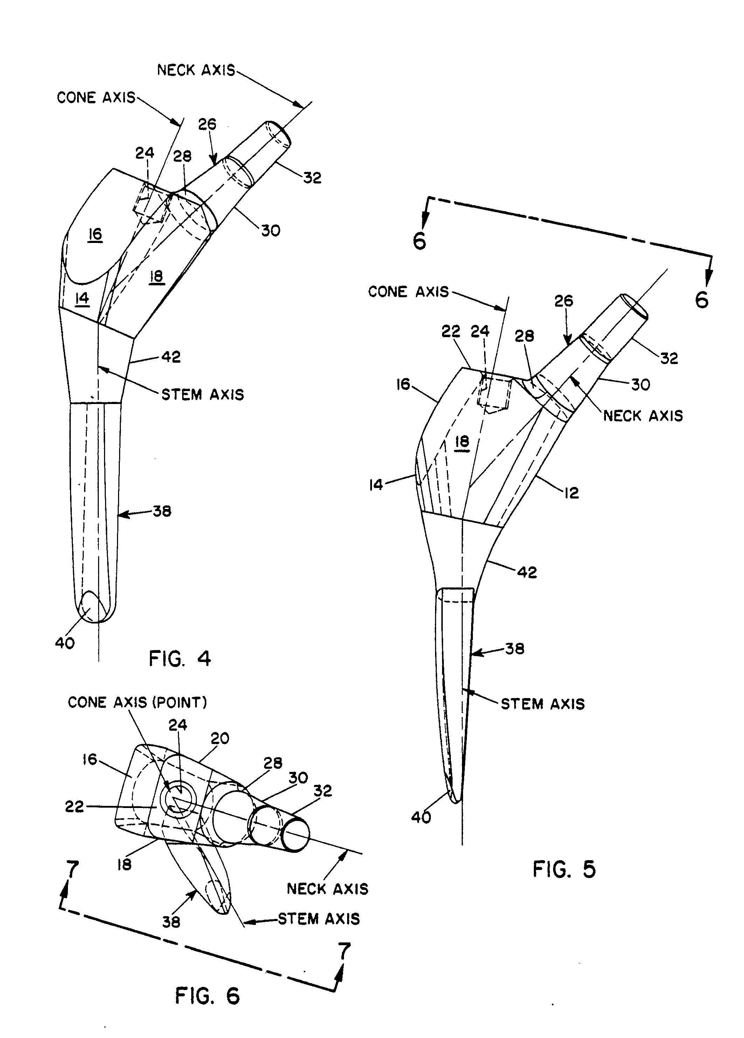

For purposes of computer-assisted design and computer-assisted manufacture (CAD-CAM) of the embodiment, the proximal portion 10 of the embodiment is based on a cone having an axis ("cone axis") and a cone angle or taper established by the medial extremity of a curved (radiused) medical aspect 12 of the proximal portion (see Figs. 7, 7A and 7B). Apart from the medial extremity, which is an element of the imaginary cone, and a vestigial conical surface 14 at the inferior, lateral extremity, the proximal portion 10 bears little resemblance to a cone. The superior lateral aspect 16 of the proximal portion 10 is a segment of a cylindrical surface generated by a line rotated about an axis located medially of the component and perpendicular to a plane defined by the neck and cone axes (see Fig. 7). The anterior and posterior aspects 18 and 20 of the proximal portion 10 are planar and lie symmetrically with respect to the cone axis and converge both medially and inferiorly (see Figs. 6, 7A and 7B). Hence, the proximal portion is a wedge-shaped body having a narrower end (the medial aspect 12) presented medially and somewhat inferiorly. The lateral portion 22 of the superior surface is flat and perpendicular to the cone axis. A threaded hole 24 opening at the surface 22 accepts an instrument used for inserting as well as for extracting the component, should revision of the hip replacement ever be required.

A neck portion 26 extends superiorly and obliquely both medially and anteriorly from the medial part of the proximal portion, beginning at a radiused and blended juncture 28 with the proximal portion 10, continuing along a conical part 30 and terminating in a Morse-type taper 32 that can accept a femoral head element (not shown) of a desired size and length driven home on the taper 32.

A stem portion 38 extends inferiorly from the proximal portion. The stem axis is oblique at a large obtuse included angle (about 160 degrees) to the cone axis (Fig. 4). It tapers inferiorly to a slender distal tip 40 from a smoothly blended juncture 42 with the proximal portion 10.

The cross-hatching in Fig. 7 represents a recess 44 that extends continuously along approximately the medial one-half of the anterior and posterior aspects 18 and 20 and across the medial aspect 12 of the proximal portion 10. The recess contains a porous medium capable of accepting an ingrowth of bone for biological retention. The technology of porous materials is known per se in the art from, for example, Morscher, E. (ed.), The Cementless Fixation of Hip Endoprostheses, New York, Springer-Verlag, 1983, and Engh, C.A., Bobyn, J.D., Biological Fixation in Total Hip Arthroplasty, Thorofare, N.J., Slack, Inc., 1985. The configuration of the prosthesis of the present invention does not depend on bone ingrowth for stability, but ingrowth should occur and enhance stability.

The preferred material for the implant is titanium alloy, Ti-6Al-4V, and the porous medium is sintered titanium beads in a recess 1,02 mm (0.40 inch) deep to a thickness of 1,27 mm (0.050 inch) plus or minus 0,25mm (0.010 inch).

The femur is prepared for reception of the implant by first reaming the femoral canal and then broaching the proximal part of the femur with a broach that is properly undersized for slight interference between the proximal portion of the component and the broached endosteal surface of the cortical bone in the region of the calcar. Except for being slightly undersize, the shape of the broached portion exactly matches the shape of the wedge-like body of the proximal portion of the prosthesis. The broaching instruments include a guide that provides for straight-line broaching along an axis corresponding to the cone axis of the component. In this respect, also, the cone axis has significance (beyond CAD-CAM, as mentioned), notwithstanding only minimal existence of actual conical surfaces in the proximal portion.

In the prosthetic joint the medial, anterior and posterior aspects of the proximal portion 10 of the femoral prothesis, according to the invention, transmit a compressive load into the proximal medial portion of the femur in the region of the calcar, where the bone is thick, strong, and heavily loaded in compression is a normal anatomical hip. When the femur bends, lateral loads may be transmitted to the lateral distal aspect of the stem portion, causing the stem to be bent and stressed. Axial stresses in the bone, however, are not transferred to the stem, so the bone is not relieved from axial loads and should retain its structural integrity. The tension loads in the femur in the region of the greater trochanter are also little affected by the prosthesis. Apart from the lateral distal tip of the stem and the region generally corresponding to the porous-coated recess, load transfer from the prosthesis to the bone is minimized.

## Claims

1. A femoral component for a hip joint prosthesis adapted to be implanted in the proximal portion of a femur for biological retention without a cement having
   - a proximal portion (10) adapted to be received in the intertrochanteric region of a femur,
   - a neck portion (26) having a distal portion that is a surface of revolution about a neck axis which extends obliquely medially and superiorly from the proximal portion (10) and is adapted to carry a femoral head element,
   - and a stem portion (38) smoothly merging with and extending inferiorly from the proximal portion (10) and having a nominal axis defined by the anterior-posterior and medial-lateral planes of the component, characterized

   - in that the proximal portion (10) has a medial aspect (12) oriented obliquely to the stem portion both anteriorly and medially that is a segment of a conical surface having a cone axis as its axis and planar anterior and posterior aspects (18, 20) that converge medially and inferiorly, intersect the medial aspect (12) and are disposed symmetrically with respect to a plane defined by the cone axis and the neck axis,
   - in that the stem axis of the stem portion (38) intersects the cone axis of the proximal portion (10) and the cone axis intersects the neck axis at respective intersections proximate to the juncture of the stem portion (38) and the proximal portion (10)
   - in that the axis of the neck portion (26) is oriented obliquely anteriorly with respect to the stem axis and obliquely medially with respect to a plane containing the stem axis and the cone axis,
   - in that the cone axis of the proximal portion (10) is oriented obliquely medially with respect to the stem axis at an angle less than that of the neck axis and obliquely anteriorly with respect to the stem axis at an angle that is also less than that of the neck axis,
   - said medial, anterior and posterior aspects (12, 18, 20) defining a generally wedge-shaped body adapted to engage the endosteal surfaces of the proximal medial portion of the femur in the intertrochanteric region such that compressive loads are transmitted from the component to the bone in the intertrochanteric region and load transfers through other surfaces are minimized.

2. A component according to claim 1, characterized in that there is a recess (44) extending continuously along the medial portions of the anterior and posterior aspects (18, 20) and across the medial aspects (12), and the recess (44) contains a porous medium adapted to accept bone ingrowth at the calcar region of the femur for biological retention.

## Revendications

1. Elément fémoral pour une prothèse d'articulation de la hanche, capable d'être implanté dans la partie proximale d'un fémur, en vue d'obtenir une prise biologique sans application de ciment, ledit élément comportant

- une partie proximale (10), capable d'être logée dans la région intertrochantérienne d'un fémur,
- un col (26) ayant une partie distale qui est une surface de révolution autour d'une axe du col qui est incliné en direction médiale et s'étend au-dessus de la partie proximale (10) et est capable de porter un élément de tête fémorale,
- et une tige (38) formant une transition progressive avec la partie proximale (10) et s'étendant en dessous de cette dernière et ayant un axe nominal défini par les plans antéropostérieur et médial-latéral de l'élément,

caractérisé

- en ce que la partie proximale (10) comprend une face médiale (12) d'orientation inclinée contre la tige aussi bien dans la direction antérieure que dans la direction médiale, ladite face étant un secteur d'une surface conique ayant comme son axe un axe de cône et des faces antérieure et postérieure planes (18, 20) qui convergent en direction médiale et vers le bas, coupent la face médiale (12) et sont disposées symétriquement par rapport à un plan défini par l'axe du cône et l'axe du col,
- en ce que l'axe de la tige (38) coupe l'axe du cône de la partie proximale (10) et l'axe du cône coupe l'axe du col respectivement à des points d'intersection situés à proximité de la jointure de la tige (38) et de la partie proximale (10),
- en ce que l'axe du col (26) est incliné vers la face antérieure par rapport à l'axe de la tige et est incliné vers la face médiale par rapport à un plan passant par l'axe de la tige et l'axe du cône,
- en ce que l'axe du cône de la partie proximale (10) est incliné vers la face médiale par rapport à l'axe de la tige sous un angle inférieur à celui de l'axe du col et est incliné vers la face antérieure par rapport à l'axe de la tige sous un angle également inférieur à celui de l'axe du col,
- en ce que les faces médiale, antérieure et postérieure (12, 18, 20) définissent un corps sensiblement en forme de coin, capable de prendre appui contre les surfaces de l'endoste de la partie proximale médiale du fémur dans la région intertrochantérienne, de manière que des charges de compression sont transmises à partir de l'élément vers l'os dans la région intertrochantérienne et que des transferts de charge à travers d'autres surfaces sont réduits au minimum.

2. Elément selon la revendication 1, caractérisé en ce qu'il est prévu une cavité (44) qui s'étend en continu le long des parties médiales des faces antérieure et postérieure (18, 20) et à travers le côté médial (12), et en ce que la cavité (44) contient un milieu poreux capable de permettre la pénétration de l'os au droit de la région calcariforme du fémur en vue d'une prise biologique.

## Patentansprüche

1. Femoralkomponente für eine Hüftgelenkprothese, die in den proximalen Abschnitt eines Femurs zum biologischen Rückhalt zementfrei implantierbar ist, mit

- einem proximalen Abschnitt (10), der im intertrochanterischen Bereich eines Femurs aufnehmbar ist,
- einem Halsabschnitt (26) mit einen distalen Abschnitt, der eine Rotationsfläche um eine Halsachse ist, der sich medial und vom proximalen Abschnitt (10) nach oben schräg erstreckt und ein Femurkopfelement zu tragen vermag,
- und einem Schaftabschnitt (38), der allmählich in den proximalen Abschnitt (10) übergeht und sich unterhalb desselben erstreckt und eine Nennachse aufweist, die durch die Anterior-Posterior- und Medial-Lateral-Ebenen der Komponente definiert sind,

dadurch gekennzeichnet,

- daß der proximale Abschnitt (10) eine mediale Seite (12) aufweist, die in bezug auf den Schaftabschnitt sowohl nach vorn als auch zur Körpermitte hin schräg ausgerichtet ist und Teil einer Kegelfläche ist, die als Achse eine Kegelachse und ebene Anterior- und Posteriorseiten (18, 20) aufweist, die zur Körpermitte hin und nach unten zusammenlaufen, die mediale Seite (12) schneiden und in bezug auf eine durch die Kegelachse und die Halsachse definierte Ebene symmetrisch angeordnet sind,
- daß die Schaftachse des Schaftabschnitts (38) die Kegelachse des proximalen Abschnitts (10) und die Kegelachse die Halsachse je an Schnittstellen schneiden, die nahe der Verbindungsstelle des Schaftabschnitts (38) und des proximalen Abschnitts (10) gelegen sind,

- daß die Achse des Halsabschnitts (26) in bezug auf die Schaftachse schräg nach vorn und in bezug auf eine durch die Schaft- und die Kegelachse gehende Ebene schräg zur Körpermitte hin ausgerichtet ist,

- die Kegelachse des proximalen Abschnitts (10) in bezug auf die Schaftachse unter einem Winkel kleiner als der Winkel der Halsachse schräg zur Körpermitte hin und in bezug auf die Schaftachse unter einem Winkel ebenfalls kleiner als der Winkel der Halsachse schräg nach vorn ausgerichtet ist,

- daß die Medial-, Anterior- und Posteriorseiten (12, 18, 20) einen zumindest annähernd keilförmigen Körper begrenzen, der sich an die endostealen Flächen des proximalen Medialabschnitts des Femurs im intertrochanterischen Bereich so anzulegen vermag, daß Drucklasten von der Komponente auf den Knochen im intertrochanterischen Bereich weitergeleitet werden und Lastübertragungen an anderen Flächen so gering wie möglich gehalten sind.

2. Komponente nach Anspruch 1, dadurch gekennzeichnet, daß sich über die medialen Abschnitte der Anterior- und Posteriorseiten (18, 20) und über die mediale Seite (12) eine Vertiefung (44) kontinuierlich erstreckt, und die Vertiefung (44) einen porösen Stoff enthält, der zum biologischen Rückhalt das Einwachsen des Knochens im Spornbereich des Femurs ermöglicht.

FIG. IA

FIG. IB

FIG. IC

FIG. I

FIG. 3

FIG. 2

NECK AXIS

CONE AXIS

26

24 28

32

16

30

18

14

42

STEM AXIS

38

40

FIG. 4

CONE AXIS (POINT)

24 20

16

28

30

32

22

18

38

NECK AXIS

STEM AXIS

7

7

FIG. 6

6

6

CONE AXIS

26

22 24

28

32

16

30

NECK AXIS

18

14

12

42

38

STEM AXIS

40

FIG. 5

8

FIG. 7

FIG. 7A

FIG. 7B